# EUROPEAN PATENT SPECIFICATION

(11) **EP 4 321 501 B1**
(45) Date of publication and mention of the grant of the patent: **01.10.2025**
(21) Application number: 22938726.1
(22) Date of filing: 27.12.2022
(51) Int. Cl.: C07C 67/347, C07C 69/716, B01J 31/02

(54) **METHOD FOR SYNTHESIZING DIMETHYL 3-(3-OXO-2-PENTYL)CYCLOPENTYLMALONATE AND CATALYST**
SYNTHETISCHES VERFAHREN UND KATALYSATOR FÜR 3-(3OXO-2-PENTYL)CYCLOPENTYLDIMETHYLMALONAT
PROCÉDÉ DE SYNTHÈSE DE 3-(3-OXO-2-PENTYL)CYCLOPENTYLMALONATE DE DIMÉTHYLE ET CATALYSEUR

(30) Priority: 11.05.2022 CN 202210513694
(43) Date of publication of application: 14.02.2024
(73) Proprietor: Zhejiang Xinhua Chemical Co., Ltd., Zhejiang 311600 (CN); Ningxia Xinhua Chemical Co., Ltd., Yinchuan, Ningxia 312500 (CN)
(72) Inventor: ZHU, Jian, Jiande, Zhejiang 311600 (CN); YING, Sibin, Jiande, Zhejiang 311600 (CN); WU, Jingheng, Jiande, Zhejiang 311600 (CN); YING, Dengyu, Jiande, Zhejiang 311600 (CN); LUO, Gongyu, Jiande, Zhejiang 311600 (CN); QIAO, Yuanyuan, Jiande, Zhejiang 311600 (CN)
(74) Representative: Cabinet Bleger-Rhein-Poupon
(86) International application number: PCT/CN2022/142370
(87) International publication number: WO 2023/216615

(56) References cited:
- EP-A1- 1 498 407
- CN-A- 101 429 122
- CN-A- 102 391 060
- CN-A- 102 391 060
- CN-A- 111 646 899
- CN-A- 114 920 648
- US-A1- 2012 004 424
- YING ANGUO ET AL: "Michael Addition of Methylene Active Compounds to Chalcones with Novel Task-specific Ionic Liquids as Catalysts under Solvent-free Conditions", CHINESE JOURNAL OF ORGANIC CHEMISTRY, vol. 31, no. 8, 1 August 2011 (2011-08-01), CN, pages 1312 - 1318, XP093107115, ISSN: 0253-2786
- YING A G ET AL: "Aza-Michael addition of aliphatic or aromatic amines to @a,@b-unsaturated compounds catalyzed by a DBU-derived ionic liquid under solvent-free conditions", TETRAHEDRON LETTERS, ELSEVIER, AMSTERDAM, NL, vol. 50, no. 14, 8 April 2009 (2009-04-08), pages 1653 - 1657, XP025950768, ISSN: 0040-4039, [retrieved on 20090129], DOI: 10.1016/J.TETLET.2009.01.123
- YING ANGUO, ZHENG,RENHUA WU,CHENGLIN LIANG,HUADING GE,CHANGHUA JIANG: "Michael Addition of Methylene Active Compounds to Chalcones with Novel Task-specific Ionic Liquids as Catalysts under Solvent-free Conditions", CHINESE JOURNAL OF ORGANIC CHEMISTRY, SCIENCE PRESS, BEIJING., CN, vol. 31, no. 8, 1 August 2011 (2011-08-01), CN , pages 1312 - 1318, XP093107115, ISSN: 0253-2786
- MICHAEL, SHUO LIU, ANGUO YING, YUXIANG NI, JIANGUO YANG, SONGLIN XU, , : "Application of Task-Specific Ionic Liquids to Michael Additions", PROGRESS IN CHEMISTRY., vol. 25, no. 8, 24 August 2013 (2013-08-24), pages 1313 - 1324, XP093107116, DOI: 1005-281X( 2013) 08-1313-12
- ZHAO, YAJUAN: "Synthesis of Methyl-Dihydrojasmonate", SHIYOU HUAGONG GAODENG XUEXIAO XUEBAO, LIAONING SHIYOU HUAGONG DAXUE, CN, vol. 10, no. 01, 25 March 1997 (1997-03-25), CN , pages 17 - 20, XP009550347, ISSN: 1006-396X
- YING, A.G. ; LIU, L. ; WU, G.F. ; CHEN, G. ; CHEN, X.Z. ; YE, W.D.: "Aza-Michael addition of aliphatic or aromatic amines to @a,@b-unsaturated compounds catalyzed by a DBU-derived ionic liquid under solvent-free conditions", TETRAHEDRON LETTERS, ELSEVIER, AMSTERDAM , NL, vol. 50, no. 14, 8 April 2009 (2009-04-08), Amsterdam , NL , pages 1653 - 1657, XP025950768, ISSN: 0040-4039, DOI: 10.1016/j.tetlet.2009.01.123
- MASE, N. ET AL.: "Organocatalytic Enantioselective Michael Additions of Malonates to 2-Cyclopentenone.", SYNLETT, no. 15, 27 July 2010 (2010-07-27), ISSN: 0936-5214

## Description

### Field of the Invention

The present disclosure relates to a synthetic method and catalyst for preparing 3-(3-oxo-2-pentyl)cyclopentyl dimethyl malonate.

### Background of the Invention

3-oxo-2-pentyl cyclopentyl methyl acetate, also known as methyl dihydrojasmonate (MDJ), belongs to jasmonate and is an important artificial flavor. MDJ is a colorless or yellowish transparent liquid with relatively stable chemical properties, and has the advantages of slow volatilization, long fragrance retention time, and will not cause discoloration when used for flavoring, and it is often used to prepare oriental flavors such as jasmine, lily of the valley, tuberosa, etc..

In industry, cyclopentanone, n-pentanal and dimethyl malonate are mainly used as raw materials to synthesize methyl dihydrojasmonate. The specific process is as follows: 1) Cyclopentanone and n-pentanal undergo condensation dehydration reaction to produce 2-pentylidene cyclopentan-1-one; 2) 2-pentylidene cyclopentan-1-one undergoes isomerization reaction to produce 2-pentyl-2-cyclopentenone; 3) 2-pentyl-2-cyclopentenone and dimethyl malonate undergo Michael Addition reaction to produce 3-(3-oxo-2-pentyl)cyclopentyl dimethyl malonate; 4) 3-(3-oxo-2-amyl)cyclopentyl dimethyl malonate is hydrolyzed and decarboxylated to give methyl dihydrojasmonate.

For the Michael Addition reaction between 2-pentyl-2-cyclopentenone and dimethyl malonate to produce 3-(3-oxo-2-pentyl)cyclopentyl dimethyl malonate, sodium methoxide is mainly used as the catalyst in industry at present, for example, Chinese patent CN101429122B discloses a decarboxylation method in the synthesis of methyl dihydrojasmonate, in which methyl dihydrojasmonate is obtained by addition and decarboxylation of 2-pentyl cyclopentenone and dimethyl malonate under the catalysis of sodium methoxide in methanol solution. However, the use of sodium methoxide as a catalyst has the following problems: first, sodium methoxide cannot be recycled and reused, acid quenching is required at the end of the reaction, and a large amount of saturated sodium bicarbonate aqueous solution and salt solution are required to wash the organic phase, which will form a large amount of salt-containing wastewater, leading to unfriendly environment; secondly, sodium methoxide is particularly sensitive to water during use, a small amount of water will cause the decomposition of sodium methoxide, leading to unstable reaction; finally, due to the high viscosity of sodium methoxide, a large amount of methanol needs to be added as the solvent in the reaction process, and the addition of solvent will not only increase the separation energy consumption, but also increase the handling load of the three wastes.

All of YING ANGUO ET AL: "Michael Addition of Methylene Active Compounds to Chalcones with Novel Task-specific lonic Liquids as Catalysts under Solvent-free Conditions" (CHINESE JOURNAL OF ORGANIC CHEMISTRY, vol.31, no.8, 1 August 2011 (2011-08-01), pages 1312-1318); CN102391060A, and YING A G ET AL: "Aza-Michael addition of aliphatic or aromatic amines to @a,@b-unsaturated compounds catalyzed by a DBU-derived ionic liquid under solvent-free conditions" (TETRAHEDRON LETTERS, ELSEVIER, AMSTERDAM, NL, vol.50, no.14, 8 April 2009(2009-04-08), pages 1653-1657) disclose an ionic liquid catalyst, however, its reaction substrate is different.

EP1498407A1 discloses a reaction of 2-pentyl-2-cyclopentenone and dimethyl malonate to form 3-(3-oxo-2-pentyl)cyclopentyl dimethyl malonate, however, it is silent on the reaction catalyst.

### Summary of the Invention

In view of the shortcomings and deficiencies of the prior art, the present disclosure provides an improved synthetic method for the preparation of of 3-(3-oxo-2-pentyl)cyclopentyl dimethyl malonate, which is environmentally friendly, stable in reaction and low in production cost.

To achieve the above purposes, a technical solution employed by the present disclosure is as follows:
A synthetic method for the preparation of 3-(3-oxo-2-pentyl)cyclopentyl dimethyl malonate, which using 2-pentyl-2-cyclopentenone and dimethyl malonate as reactants, and reacting the reactants in the presence of a catalyst and a monodentate phosphine ligand to prepare 3-(3-oxo-2-pentyl)cyclopentyl dimethyl malonate, the catalyst is a basic ionic liquid, the pH value of the basic ionic liquid is greater than or equal to 10; and the catalyst is prepared by a preparation method comprising the following steps of: mixing the nitrogen-containing heterocyclic compound with an aliphatic carboxylate or hydroxyl aliphatic carboxylate or fluorophosphate under stirring;
the aliphatic carboxylate is selected from the group consisting of the salt of R₁COOH or HOOCR₂COOH, and the hydroxyl aliphatic carboxylate is the salt of OHR₃COOH, and the fluorophosphates is selected from the group consisting of trifluorophosphate, tetrafluorophosphate, hexafluorophosphate, and combinations thereof; wherein, R₁ is selected from the group consisting of C1 - C8 alkyl, R₂ is selected from the group consisting of a single bond, and C1 - C7 alkylidene, and R₃ is selected from the group consisting of C1 - C5 alkylidene;
the nitrogen-containing heterocyclic compound is selected from the group consisting of 1,8-diazabicyclo[5.4.0]undec-7-ene 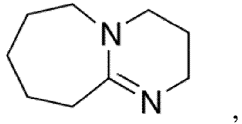 4-dimethylaminopyridine 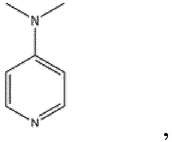 1,5-diazabicyclo[4.3.0]non-5-ene 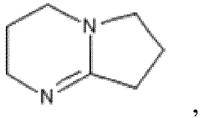 and combinations thereof.

The presence of the monodentate phosphine ligand can further improve the conversion of 2-pentyl-2-cyclopentenone.

In some implementations of the present disclosure, the pH value of the basic ionic liquid is 12 - 14.

In some implementations of the present disclosure, the mixing temperature for the catalyst preparation is 20 - 80°C, and the mixing time is 4 - 24 h.

In some implementations of the present disclosure, the aliphatic carboxylate is selected from the group consisting of acetate, propionate or oxalate; and the hydroxyl aliphatic carboxylate is selected from the group consisting of glycolate, hydroxypropionate or hydroxybutyrate.

Michael Addition reaction between 2-pentyl-2-cyclopentenone and dimethyl malonate uder the action of the basic ionic liquid generates 3-(3-oxo-2-pentyl)cyclopentyl dimethyl malonate, with the reaction formula as follows:

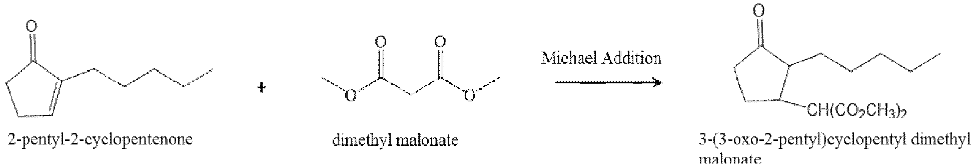

The inventor found through research that when a strong basic ionic liquid with a pH value greater than or equal to 10 formed by nitrogen-containing heterocyclic compound is used as the catalyst for Michael Addition reaction between 2-pentyl-2-cyclopentenone and dimethyl malonate to generate 3-(3-oxo-2-pentyl)cyclopentyl dimethyl malonate, the reaction does not need solvents, and the reaction can be environmentally friendly, stable and low in production cost.

Further, the monodentate phosphine ligand is selected from the group consisting of triphenylphosphine, [4-(N,N-dimethylamino)phenyl]di(tert-butyl)phosphine, diphenyl-2-pyridylphosphine, [4-(dimethylamino)phenyl]diphenylphosphine, combinations thereof.

Preferably, the molar ratio of the monodentate phosphine ligand to the catalyst is 1: (1 - 50).

In some implementations of the present disclosure, the molar ratio of 2-pentyl-2-cyclopentenone to dimethyl malonate is 1: (0.5 - 5).

In some implementations of the present disclosure, the mass ratio of the catalyst to dimethyl malonate is 1: (10 - 50).

In some implementations of the present disclosure, the synthetic method comprises steps of: mixing dimethyl malonate, the catalyst and the monodentate phosphine ligand to obtain a mixture, adding 2-pentyl-2-cyclopentenone dropwise into the mixture, and stirring at a constant temperature to continue the reaction after the addition.

Further, the temperature of the mixture during addition is -10~30°C, and the addition time is 1~10 h.

Further, the constant temperature is -10~50°C, and the time for the constant temperature is 1~30 h.

Preferably, after the addition is completed, the reaction system is cooled to - 5°C, and then stirred to continue the reaction at this temperature.

In some implementations of the present disclosure, the synthetic method further comprises steps of adding water to the reaction system for static stratification after the reaction is completed.

After static stratification, the upper layer is an organic layer and the lower layer is a water layer, the water in the water layer can be removed by distillation to recover the basic ionic liquid catalyst.

The present disclosure has the following advantages over the prior art:
The mixing between the nitrogen-containing heterocyclic compound with the aliphatic carboxylate or hydroxyl aliphatic carboxylate or fluorophosphate is used to form a strong basic ionic liquid with a pH value greater than or equal to 10, and when it is used to as catalyst for the synthetic method, the synthetic method does not need solvents, the reaction conditions are mild, and the conversion rate of 2-pentyl-2-cyclopentenone can be improved.

The catalyst of the present disclosure is simple to prepare, easy to recycle and reuse, and remains basically unchanged in activity after being applied multiple times, and has good stability.

The synthetic method of the present disclosure does not require solvents, and only water is used for catalyst recycle, resulting in low discharge of three wastes, low cost, and environmental friendliness.

By adding the monodentate phosphine ligand in the reaction system, the present disclosure can further improve the conversion rate of 2-pentyl-2-cyclopentenone, which is up to 99%.

### Detailed Description of Exemplary Embodiments

The present disclosure is further described below combining with embodiments. But the present disclosure is not limited to the embodiments below. The implementation conditions used in the embodiments may be further adjusted according to different requirements of specific use, and undefined implementation conditions usually are conventional conditions in the industry. The technical features involved in the various embodiments of the present disclosure may be combined with each other if they do not conflict with each other.

### Reference Embodiment 1

### 1) Preparation of basic ionic liquid

0.5 mol of 1,8-diazabicyclo[5.4.0]undec-7-ene was weighed, 1 mol of sodium acetate was placed in a beaker, and stirred at 30°C for 6 h at a constant temperature, to give the desired ionic liquid with a pH value of 13.2.

### 2) Preparation of 3-(3-oxo-2-pentyl)cyclopentyl dimethyl malonate

2 g of the above ionic liquid and 50 g of dimethyl malonate were weighed into a 100 mL three necked flask, the three necked flask was placed in a 25°C constant-temperature water bath; then 56 g of 2-pentyl-2-cyclopentenone was slowly added dropwise, the addition time was controlled to be to 6 h, and after the dropwise addition was completed, the temperature of the water bath was lowered to -5°C, and the system was stirred for 6 h at a constant temperature.

### 3) Product analysis

After the reaction was completed, 10 g of deionized water was added, and the system was stirred thoroughly and static stratification. The upper layer was the organic phase and the lower layer was the aqueous solution of ionic liquid. The products were analyzed using an Agilent 7890 gas chromatograph, in which the column was HP-INNOWax and the detector was a TCD detector. The conversion rate and selectivity were calculated by normalization.

### Reference Embodiment 2

It was basically the same as Embodiment 1, differing only in that 1,8-diazabicyclo[5.4.0]undec-7-ene was replaced by 4-dimethylaminopyridine, and the pH value of the ionic liquid was 11.7.

### Reference Embodiment 3

It was basically the same as Embodiment 1, differing only in that 1,8-diazabicyclo[5.4.0]undec-7-ene was replaced by 1,5-diazabicyclo[4.3.0]non-5-ene, and the pH value of the ionic liquid was 13.4.

For Embodiments 1 - 3, the conversion rate of 2-pentyl-2-cyclopentenone and the selectivity of 3-(3-oxo-2-pentyl)cyclopentyl dimethyl malonate are shown in Table 1 below:

**Table 1 Conversion rate and selectivity of Embodiments 1 - 3**

| Embodiments | Nitrogen-containing heterocyclic compounds | Conversion rate of 2-pentyl-2-cyclopentenone (%) | Selectivity of 3-(3-oxo-2-pentyl)cyclopentyl dimethyl malonate (%) |
|---|---|---|---|
| Embodiment 1 | 1,8-diazabicyclo[5.4.0]undec-7-ene | 93 | 98 |
| Embodiment 2 | 4-dimethylaminopyridine | 91 | 97 |
| Embodiment 3 | 1,5-diazabicyclo[4.3.0]non-5-ene | 93 | 96 |

As can be seen from Table 1, the different nitrogen-containing heterocyclic basic ionic liquids showed excellent activity, with a conversion rate of 2-pentyl-2-cyclopentenone ≥ 90% and a selectivity of 3-(3-oxo-2-pentyl)cyclopentyl dimethyl malonate > 95%.

### Reference Embodiment 4

It was basically the same as Embodiment 1, differing only in that sodium acetate was replaced by potassium acetate, and the pH value of the ionic liquid was 13.3.

### Reference Embodiment 5

It was basically the same as Embodiment 1, differing only in that sodium acetate was replaced by lithium acetate, and the pH value of the ionic liquid was 12.9.

For Embodiments 1, 4 - 5, the conversion rate of 2-pentyl-2-cyclopentenone and the selectivity of 3-(3-oxo-2-pentyl)cyclopentyl dimethyl malonate are shown in Table 2 below:

**Table 2 Conversion rate and selectivity of Embodiments 1, 4 - 5**

| Embodiments | Aliphatic carboxylates | Conversion rate of 2-pentyl-2-cyclopentenone (%) | Selectivity of 3-(3-oxo-2-pentyl)cyclopentyl dimethyl malonate (%) |
|---|---|---|---|
| Embodiment 1 | Sodium acetate | 93 | 98 |
| Embodiment 4 | Potassium acetate | 92 | 97 |
| Embodiment 5 | Lithium acetate | 93 | 96 |

As can be seen from Table 2, the three cations, namely sodium, potassium and lithium, resulted in similar activity of nitrogen-containing heterocyclic basic ionic liquids, with a conversion rate of 2-pentyl-2-cyclopentenone > 90% and a selectivity of 3-(3-oxo-2-pentyl)cyclopentyl dimethyl malonate > 95%.

### Reference Embodiments 6 - 13

It was basically the same as Embodiment 1, differing only in that sodium acetate was replaced by aliphatic carboxylates or hydroxyl aliphatic carboxylates or fluorophosphates showed in Table 3. For Embodiments 6 - 13, the conversion rate of 2-pentyl-2-cyclopentenone and the selectivity of 3-(3-oxo-2-pentyl)cyclopentyl dimethyl malonate are shown in Table 3 below:

**Table 3 Conversion rate and selectivity of Embodiments 1, 6 - 13**

| Embodiments | Aliphatic carboxylates or hydroxyl aliphatic carboxylates or fluorophosphates | pH of basic ionic liquids | Conversion rate of 2-pentyl-2-cyclopentenone (%) | Selectivity of 3-(3-oxo-2-pentyl)cyclopentyl dimethyl malonate (%) |
|---|---|---|---|---|
| Embodiment 1 | Sodium acetate | 13.2 | 93 | 98 |
| Embodiment 6 | Sodium propionate | 12.4 | 93 | 97 |
| Embodiment 7 | Sodium oxalate | 11.9 | 92 | 98 |
| Embodiment 8 | Sodium glycolate | 11.7 | 88 | 97 |
| Embodiment 9 | Sodium hydroxypropionate | 12.4 | 90 | 97 |
| Embodiment 10 | Sodium hydroxybutyrate | 11.9 | 87 | 96 |
| Embodiment 11 | Sodium trifluorophosphate | 13.1 | 93 | 95 |
| Embodiment 12 | Sodium tetrafluorophosphate | 13.1 | 92 | 93 |
| Embodiment 13 | Sodium hexafluorophosphate | 12.7 | 92 | 93 |

As can be seen from Table 3, all of the basic ionic liquids prepared with different anions in the selected range showed excellent catalytic activity, with a conversion rate of 2-pentyl-2-cyclopentenone > 88% and a selectivity of 3-(3-oxo-2-pentyl)cyclopentyl dimethyl malonate > 90%.

### Reference Embodiment 14

The aqueous solution of the ionic liquid in Embodiment 1 was rotary evaporated to remove the water, and the ionic liquid can be recycled, the reusing method being the same as in Embodiment 1. The recycle results are shown in Table 4:

**Table 4 Comparison of the performance of recycled basic ionic liquids prepared in Embodiment 1**

| Recycled times | Conversion rate of 2-pentyl-2-cyclopentenone (%) | Selectivity of 3-(3-oxo-2-pentyl)cyclopentyl dimethyl malonate (%) |
|---|---|---|
| 1 | 93 | 98 |
| 2 | 93 | 96 |
| 3 | 94 | 98 |
| 4 | 93 | 97 |
| 5 | 92 | 98 |

As can be seen from Table 4, after being recycled for 5 times, the activity of the catalyst remains basically unchanged, indicating that the performance of the prepared basic ionic liquid is stable.

### Embodiment 15

### 1) Preparation of basic ionic liquid

The preparation method was the same as in Embodiment 1.

### 2) Preparation of 3-(3-oxo-2-pentyl)cyclopentyl dimethyl malonate

2 g of the above ionic liquid, 0.3 g of [4-(N,N-dimethylamino)phenyl]di(tert-butyl)phosphine, and 50 g of dimethyl malonate were weighed into a 100 mL three necked flask, the three necked flask was placed in a 25°C constant-temperature water bath; then 56 g of 2-pentyl-2-cyclopentenone was slowly added dropwise, the addition time was controlled to be to 6 h, and after the dropwise addition was completed, the temperature of the water bath was lowered to -5°C, and the system was stirred for 6 h at a constant temperature.

### 3) Product analysis

The analysis method was the same as in Embodiment 1.

### Embodiments 16 - 18

They were basically the same as Embodiment 15, differing only in that [4-(N,N-dimethylamino)phenyl]di(tert-butyl)phosphine was replaced by triphenylphosphine, diphenyl-2-pyridylphosphine, [4-(dimethylamino)phenyl]diphenylphosphine, respectively. The catalytic performance of Embodiments 15 - 18 is shown in Table 5 below:

**Table 5 Conversion rate and selectivity of Embodiments 1, 15 - 18**

| Embodiments | Monodentate phosphine ligands | Conversion rate of 2-pentyl-2-cyclopentenone (%) | Selectivity of 3-(3-oxo-2-pentyl)cyclopentyl dimethyl malonate (%) |
|---|---|---|---|
| Embodiment 1 | None | 93 | 98 |
| Embodiment 15 | [4-(N,N-dimethylamino)phenyl]di(ter t-butyl)phosphine | 99 | 98 |
| Embodiment 16 | Triphenylphosphine | 95 | 96 |
| Embodiment 17 | Diphenyl-2-pyridylphosphine | 97 | 96 |
| Embodiment 18 | [4-(dimethylamino)phenyl]diph enylphosphine | 99 | 97 |

As can be seen from Table 5, the addition of the monodentate phosphine ligands can increase the conversion rate of 2-pentyl-2-cyclopentenone, with a conversion rate of 2-pentyl-2-cyclopentenone > 95% and a selectivity of 3-(3-oxo-2-pentyl)cyclopentyl dimethyl malonate > 95%.

### Comparative example 1

It was basically the same as Embodiment 1, differing only in that: the 1,8-diazabicyclo[5.4.0]undec-7-ene was replaced by methylimidazole, and the pH value of the ionic liquid was 8.7.

The results showed that the conversion rate of 2-pentyl-2-cyclopentenone was 41%, and the selectivity of 3-(3-oxo-2-pentyl)cyclopentyl dimethyl malonate was 81%.

### Comparative example 2

1) 2 g of 1,8-diazabicyclo[5.4.0]undec-7-ene and 50 g of dimethyl malonate were weighed into a 100 mL three necked flask, the three necked flask was placed in a 25°C constant-temperature water bath; then 56 g of 2-pentyl-2-cyclopentenone was slowly added dropwise, the addition time was controlled to be to 6 h, and after the addition was completed, the temperature of the water bath was lowered to -5°C, and the system was stirred for 6 h at a constant temperature.
2) After the reaction was completed, 10 g of deionized water was added, and the system was stirred thoroughly and static stratification. The upper layer was the organic phase, and the product was analyzed by gas chromatography.

### Comparative examples 3 - 4

They were basically the same as Embodiment 2, differing only in that: 1,8-diazabicyclo[5.4.0]undec-7-ene was replaced by 4-dimethylaminopyridine,1,5-diazabicyclo[4.3.0]non-5-ene, respectively. The catalytic activity results for Comparative examples 2- 4 are shown in Table 6 below:

**Table 6 Conversion rate and selectivity of Comparative examples 2- 4**

| Comparative examples | Conversion rate of 2-pentyl-2-cyclopentenone (%) | Selectivity of 3-(3-oxo-2-pentyl)cyclopentyl dimethyl malonate (%) |
|---|---|---|
| Comparative example 2 | 44 | 97 |
| Comparative example 3 | 39 | 97 |
| Comparative example 4 | 43 | 93 |

As can be seen from Table 6, nitrogen-containing heterocyclic compounds can also catalyze the reaction between dimethyl malonate and 2-pentyl-2-cyclopentenone, but the conversion rate of 2-pentyl-2-cyclopentenone is significantly reduced, indicating that the conversion rate of 2-pentyl-2-cyclopentenone can be significantly improved after nitrogen-containing heterocyclic compounds form ionic liquids with aliphatic carboxylates or hydroxyl aliphatic carboxylates or fluorophosphate.

### Comparative example 5

1) 2 g of sodium acetate, 0.3 g of [4-(N,N-dimethylamino)phenyl]di(tert-butyl)phosphine, and 50 g of dimethyl malonate were weighed into a 100 mL three necked flask, the three necked flask was placed in a 25°C constant-temperature water bath; then 56 g of 2-pentyl-2-cyclopentenone was slowly added dropwise, the addition time was controlled to be to 6 h, and after the dropwise addition was completed, the temperature of the water bath was lowered to -5°C, and the system was stirred for 6 h at a constant temperature.
2) After the reaction was completed, 10 g of deionized water was added, and the system was stirred thoroughly and static stratification. The upper layer was an organic phase, and the analysis showed that 3-(3-oxo-2-pentyl)cyclopentyl dimethyl malonate was not detected in the reactants. These results indicatd that nitrogen-containing heterocyclic compounds are the main active sites of the catalyst.

The embodiments described above are only for illustrating the technical concepts and features of the present disclosure, and are intended to make those skilled in the art being able to understand the present disclosure and thereby implement it, and should not be concluded to limit the protective scope of this disclosure.

## Claims

1. A synthetic method for the preparation of 3-(3-oxo-2-pentyl)cyclopentyl dimethyl malonate, which using 2-pentyl-2-cyclopentenone and dimethyl malonate as reactants, and reacting the reactants in the presence of a catalyst and a monodentate phosphine ligand to prepare 3-(3-oxo-2-pentyl)cyclopentyl dimethyl malonate, **characterized in that,** the catalyst is a basic ionic liquid, the pH value of the basic ionic liquid is greater than or equal to 10; and the catalyst is prepared by a preparation method comprising the following steps of: mixing the nitrogen-containing heterocyclic compound with an aliphatic carboxylate or hydroxyl aliphatic carboxylate or fluorophosphate under stirring;
the aliphatic carboxylate is selected from the group consisting of the salt of R₁COOH or HOOCR₂COOH, and the hydroxyl aliphatic carboxylate is the salt of OHR₃COOH, and the fluorophosphates is selected from the group consisting of trifluorophosphate, tetrafluorophosphate, hexafluorophosphate, and combinations thereof, wherein, R₁ is selected from the group consisting of C1 - C8 alkyl, R₂ is selected from the group consisting of a single bond, and C1 - C7 alkylidene, and R₃ is selected from the group consisting of C1 - C5 alkylidene;
the nitrogen-containing heterocyclic compound is selected from the group consisting of 1,8-diazabicyclo[5.4.0]undec-7-ene , 4-dimethylaminopyridine , 1,5-diazabicyclo[4.3.0]non-5-ene , and combinations thereof.

2. The synthetic method according to claim 1, **characterized in that,** the pH value of the basic ionic liquid is 12 - 14.

3. The synthetic method according to claim 1, **characterized in that,** for catalyst preparation the mixing temperature is 20 - 80°C, and the mixing time is 4 - 24 h.

4. The synthetic method according to claim 1, **characterized in that,** the aliphatic carboxylate is selected from the group consisting of acetate, propionate or oxalate; and the hydroxyl aliphatic carboxylate is selected from the group consisting of glycolate, hydroxypropionate or hydroxybutyrate.

5. The synthetic method according to claim 1, **characterized in that,** the monodentate phosphine ligand is selected from the group consisting of triphenylphosphine, [4-(N,N-dimethylamino)phenyl]di(tert-butyl)phosphine, diphenyl-2-pyridylphosphine, [4-(dimethylamino)phenyl]diphenylphosphine, combinations thereof.

6. The synthetic method according to claim 1, **characterized in that,** the molar ratio of the monodentate phosphine ligand to the catalyst is 1: (1 - 50).

7. The synthetic method according to claim 1, **characterized in that,** the molar ratio of 2-pentyl-2-cyclopentenone to dimethyl malonate is 1: (0.5 - 5); and/or, the mass ratio of the catalyst to dimethyl malonate is 1: (10 - 50).

8. The synthetic method according to claim 1, **characterized in that,** the pH value of the basic ionic liquid is 12 - 14, the nitrogen-containing heterocyclic compound is selected from the group consisting of 1,8-diazabicyclo[5.4.0]undec-7-ene, 4-dimethylaminopyridine, 1,5-diazabicyclo[4.3.0]non-5-ene, and combinations thereof; the aliphatic carboxylate is selected from the group consisting of acetate, propionate or oxalate; and the hydroxyl aliphatic carboxylate is selected from the group consisting of glycolate, hydroxypropionate or hydroxybutyrate; and the fluorophosphates is selected from the group consisting of trifluorophosphate, tetrafluorophosphate, hexafluorophosphate, and combinations thereof; the molar ratio of 2-pentyl-2-cyclopentenone to dimethyl malonate is 1: (0.5 - 5); the mass ratio of the catalyst to dimethyl malonate is 1: (10 - 50).

9. The synthetic method according to any one of claims 1 to 8, **characterized in that,** the synthetic method comprises steps of: mixing dimethyl malonate, the catalyst and the monodentate phosphine ligand to obtain a mixture, adding 2-pentyl-2-cyclopentenone dropwise into the mixture, and stirring at a constant temperature to continue the reaction after the addition.

10. The synthetic method according to claim 9, **characterized in that,** the temperature of the mixture during addition is -10 ~ 30°C, and the addition time is 1 ~ 10 h; and/or, the constant temperature is -10 ~ 50°C, and the time for the constant temperature is 1 ~ 30 h.

11. The synthetic method according to claim 9, **characterized in that,** the synthetic method further comprises steps of adding water to the reaction system for static stratification after the reaction is completed.

## Patentansprüche

1. Syntheseverfahren zur Herstellung von 3-(3-Oxo-2-pentyl)cyclopentyl-dimethylmalonat durch Umsetzung von 2-Pentyl-2-cyclopentenon und Dimethylmalonat als Reaktanten in Gegenwart eines Katalysators und eines monodentaten Phosphinliganden, **dadurch gekennzeichnet, dass** der Katalysator eine basische ionische Flüssigkeit ist, deren pH-Wert größer oder gleich 10 ist; und der Katalysator durch ein Herstellungsverfahren hergestellt wird, das die folgenden Schritte umfasst:
Mischen der stickstoffhaltigen heterocyclischen Verbindung mit einem aliphatischen Carboxylat oder einem hydroxyaliphatischen Carboxylat oder einem Fluorophosphat unter Rühren;
wobei das aliphatische Carboxylat ausgewählt ist aus der Gruppe bestehend aus dem Salz von R₁COOH oder HOOCR₂COOH, und das hydroxyaliphatische Carboxylat das Salz von OHR₃COOH ist, und die Fluorophosphate ausgewählt sind aus der Gruppe bestehend aus Trifluorphosphat, Tetrafluorphosphat, Hexafluorphosphat und Kombinationen davon;
wobei R₁ ausgewählt ist aus der Gruppe bestehend aus C₁-C₈-Alkyl, R₂ ausgewählt ist aus der Gruppe bestehend aus einer Einfachbindung und C₁-C₇-Alkyliden, und R₃ ausgewählt ist aus der Gruppe bestehend aus C₁-C₅-Alkyliden;
wobei die stickstoffhaltige heterocyclische Verbindung ausgewählt ist aus der Gruppe bestehend aus 1,8-Diazabicyclo[5.4.0]undec-7-en, 4-Dimethylaminopyridin, 1,5-Diazabicyclo[4.3.0]non-5-en und Kombinationen davon.

2. Syntheseverfahren nach Anspruch 1, **dadurch gekennzeichnet, dass** der pH-Wert der basischen ionischen Flüssigkeit 12 - 14 beträgt.

3. Syntheseverfahren nach Anspruch 1, **dadurch gekennzeichnet, dass** für die Katalysatorherstellung die Mischtemperatur 20 - 80 °C und die Mischzeit 4 - 24 h beträgt.

4. Syntheseverfahren nach Anspruch 1, **dadurch gekennzeichnet, dass** das aliphatische Carboxylat ausgewählt ist aus der Gruppe bestehend aus Acetat, Propionat oder Oxalat; und das hydroxyaliphatische Carboxylat ausgewählt ist aus der Gruppe bestehend aus Glycolat, Hydroxypropionat oder Hydroxybutyrat.

5. Syntheseverfahren nach Anspruch **1, dadurch gekennzeichnet, dass** der monodentate Phosphinligand ausgewählt ist aus der Gruppe bestehend aus Triphenylphosphin, [4-(N,N-Dimethylamino)phenyl]di(tert-butyl)phosphin, Diphenyl-2-pyridylphosphin, [4-(Dimethylamino)phenyl]diphenylphosphin und Kombinationen davon.

6. Syntheseverfahren nach Anspruch 1, **dadurch gekennzeichnet, dass** das Molverhältnis des monodentaten Phosphinliganden zum Katalysator 1 : (1 - 50) beträgt.

7. Syntheseverfahren nach Anspruch **1, dadurch gekennzeichnet, dass** das Molverhältnis von 2-Pentyl-2-cyclopentenon zu Dimethylmalonat 1 : (0,5 - 5) beträgt; und/oder das Massenverhältnis des Katalysators zu Dimethylmalonat 1 : (10 - 50) beträgt.

8. Syntheseverfahren nach Anspruch **1, dadurch gekennzeichnet, dass** der pH-Wert der basischen ionischen Flüssigkeit 12 - 14 beträgt, die stickstoffhaltige heterocyclische Verbindung ausgewählt ist aus der Gruppe bestehend aus 1,8-Diazabicyclo[5.4.0]undec-7-en, 4-Dimethylaminopyridin, 1,5-Diazabicyclo[4.3.0]non-5-en und Kombinationen davon; das aliphatische Carboxylat ausgewählt ist aus der Gruppe bestehend aus Acetat, Propionat oder Oxalat; und das hydroxyaliphatische Carboxylat ausgewählt ist aus der Gruppe bestehend aus Glycolat, Hydroxypropionat oder Hydroxybutyrat; und die Fluorophosphate ausgewählt sind aus der Gruppe bestehend aus Trifluorphosphat, Tetrafluorphosphat, Hexafluorphosphat und Kombinationen davon; das Molverhältnis von 2-Pentyl-2-cyclopentenon zu Dimethylmalonat 1 : (0,5 - 5) beträgt; das Massenverhältnis des Katalysators zu Dimethylmalonat 1 : (10 - 50) beträgt.

9. Syntheseverfahren nach einem der Ansprüche 1 bis 8, **dadurch gekennzeichnet, dass** das Syntheseverfahren die Schritte umfasst: Mischen von Dimethylmalonat, dem Katalysator und dem monodentaten Phosphinliganden, um eine Mischung zu erhalten, tropfenweises Zugeben von 2-Pentyl-2-cyclopentenon in die Mischung und Rühren bei einer konstanten Temperatur, um die Reaktion nach der Zugabe fortzusetzen.

10. Syntheseverfahren nach Anspruch 9, **dadurch gekennzeichnet, dass** die Temperatur der Mischung während der Zugabe -10 bis 30 °C und die Zugabezeit 1 bis 10 h beträgt; und/oder die konstante Temperatur -10 bis 50 °C und die Zeit für die konstante Temperatur 1 bis 30 h beträgt.

11. Syntheseverfahren nach Anspruch 9, **dadurch gekennzeichnet, dass** das Syntheseverfahren ferner nach Abschluss der Reaktion den Schritt des Zugebens von Wasser zum Reaktionssystem zur statischen Schichttrennung umfasst.

## Revendications

1. Procédé de synthèse pour la préparation du malonate de diméthyle de 3-(3-oxo-2-pentyl)cyclopentyle, comprenant la mise en réaction de 2-pentyl-2-cyclopenténone et de malonate de diméthyle en tant que réactifs, en présence d'un catalyseur et d'un ligand phosphine monodentate pour préparer le malonate de diméthyle de 3-(3-oxo-2-pentyl)cyclopentyle, **caractérisé en ce que** le catalyseur est un liquide ionique basique, la valeur de pH du liquide ionique basique est supérieure ou égale à 10 ; et le catalyseur est préparé par un procédé de préparation comprenant les étapes suivantes consistant à : mélanger le composé hétérocyclique azoté avec un carboxylate aliphatique ou un carboxylate aliphatique hydroxylé ou un fluorophosphate sous agitation ;
le carboxylate aliphatique est choisi dans le groupe constitué par le sel de R₁COOH ou de HOOCR₂COOH, et le carboxylate aliphatique hydroxylé est le sel de OHR₃COOH, et les fluorophosphates sont choisis dans le groupe constitué par le trifluorophosphate, le tétrafluorophosphate, l'hexafluorophosphate, et leurs combinaisons ; dans lequel, R₁ est choisi dans le groupe constitué par un alkyle en C₁-C₈, R₂ est choisi dans le groupe constitué par une liaison simple et un alkylidène en C₁-C₇, et R₃ est choisi dans le groupe constitué par un alkylidène en C₁-*C*₅ ;
le composé hétérocyclique azoté est choisi dans le groupe constitué par le 1,8-diazabicyclo[5.4.0]undéc-7-ène, la 4-diméthylaminopyridine, le 1,5-diazabicyclo[4.3.0]non-5-ène, et leurs combinaisons.

2. Procédé de synthèse selon la revendication 1, **caractérisé en ce que** la valeur de pH du liquide ionique basique est de 12 à 14.

3. Procédé de synthèse selon la revendication 1, **caractérisé en ce que**, pour la préparation du catalyseur, la température de mélange est de 20 à 80 °C, et la durée de mélange est de 4 à 24 h.

4. Procédé de synthèse selon la revendication 1, **caractérisé en ce que** le carboxylate aliphatique est choisi dans le groupe constitué par l'acétate, le propionate ou l'oxalate ; et le carboxylate aliphatique hydroxylé est choisi dans le groupe constitué par le glycolate, l'hydroxypropionate ou l'hydroxybutyrate.

5. Procédé de synthèse selon la revendication **1, caractérisé en ce que** le ligand phosphine monodentate est choisi dans le groupe constitué par la triphénylphosphine, la [4-(N,N-diméthylamino)phényl]di(tert-butyl)phosphine, la diphényl-2-pyridylphosphine, la [4-(diméthylamino)phényl]diphénylphosphine, et leurs combinaisons.

6. Procédé de synthèse selon la revendication 1, **caractérisé en ce que** le rapport molaire du ligand phosphine monodentate au catalyseur est de 1:(1 à 50).

7. Procédé de synthèse selon la revendication 1, **caractérisé en ce que** le rapport molaire de la 2-pentyl-2-cyclopenténone au malonate de diméthyle est de 1:(0,5 à 5) ; et/ou, le rapport massique du catalyseur au malonate de diméthyle est de 1:(10 à 50).

8. Procédé de synthèse selon la revendication 1, **caractérisé en ce que** la valeur de pH du liquide ionique basique est de 12 à 14, le composé hétérocyclique azoté est choisi dans le groupe constitué par le 1,8-diazabicyclo[5.4.0]undéc-7-ène, la 4-diméthylaminopyridine, le 1,5-diazabicyclo[4.3.0]non-5-ène, et leurs combinaisons ; le carboxylate aliphatique est choisi dans le groupe constitué par l'acétate, le propionate ou l'oxalate ; et le carboxylate aliphatique hydroxylé est choisi dans le groupe constitué par le glycolate, l'hydroxypropionate ou l'hydroxybutyrate ; et les fluorophosphates sont choisis dans le groupe constitué par le trifluorophosphate, le tétrafluorophosphate, l'hexafluorophosphate, et leurs combinaisons ; le rapport molaire de la 2-pentyl-2-cyclopenténone au malonate de diméthyle est de 1:(0,5 à 5) ; le rapport massique du catalyseur au malonate de diméthyle est de 1:(10 à 50).

9. Procédé de synthèse selon l'une quelconque des revendications 1 à 8, **caractérisé en ce que** le procédé de synthèse comprend les étapes consistant à : mélanger du malonate de diméthyle, le catalyseur et le ligand phosphine monodentate pour obtenir un mélange, ajouter de la 2-pentyl-2-cyclopenténone goutte à goutte dans le mélange, et agiter à une température constante pour poursuivre la réaction après l'addition.

10. Procédé de synthèse selon la revendication 9, **caractérisé en ce que** la température du mélange pendant l'addition est de -10 à 30 °C, et la durée d'addition est de 1 à 10 h ; et/ou, la température constante est de -10 à 50 °C, et la durée de maintien à la température constante est de 1 à 30 h.

11. Procédé de synthèse selon la revendication 9, **caractérisé en ce que** le procédé de synthèse comprend en outre l'étape consistant à ajouter de l'eau au système réactionnel pour une stratification statique une fois la réaction terminée.
